# EUROPEAN PATENT APPLICATION

(11) **EP 4 578 926 A1**
(43) Date of publication of application: **02.07.2025**
(21) Application number: 23220490.9
(22) Date of filing: 28.12.2023
(51) Int. Cl.: C09K 3/18, C12N 11/12

(54) **DEICING LIQUID FORMULATION**

(71) Applicant: Earth Alive Clean Technologies Inc., Montréal, Québec H2Z 1S4 (CA)
(72) Inventor: TOUSSAINT, Claudia, Montréal, H2Z 1S4 (CA); NEUFELD, Simon, Montréal, H2Z 1S4 (CA); SAFINA, Gulnaz, Montréal, H2Z 1S4 (CA)
(74) Representative: Calysta NV

(57) **Abstract**

The present invention relates to a deicing liquid formulation intended for spray application comprising glycerin, potassium formate and water. The present invention also refers to a method for deicing. The present invention finally concerns a utilization of the deicing liquid formulation of the invention. Moreover, the present invention concerns a deicing and dustsuppressant liquid formulation.

## Description

The present invention relates to a deicing liquid formulation intended for spray application.

The present invention also refers to a method for deicing.

The present invention finally concerns a utilization of the deicing liquid formulation of the invention.

Several methods of deicing already exist and comprise applying on ground deicing fluids to melt ice, snow, and frost and to prevent reformation, using unheated forced air to blow off loose snow and ice, using infrared heating to melt snow, ice and frost without using chemicals or even mechanical deicing using tools such as brooms, scrapers and ropes.

More particularly, deicers, deicing fluids, deicing liquid formulation are used for removing snow, ice or frost from a surface, a substrate. These formulations can also be complemented by an anti-icing activity of compounds on the surface/substrate to continue to delay the reformation of ice or prevent adhesion of ice.

Deicing formulations are commonly used for a large range of surfaces or substrates like, as non-limitative list, roads, roadways, highways, parking areas, walkways, bridges, cars and trucks including their windows and windscreens, trains and rail switches, aircrafts, airport pavement including runways, taxiways, airport aprons and taxiway bridges. More generally deicing formulations are used at home and for domestic use, like on sidewalks and driveways.

The working principle of deicers is by lowering the freezing point of water and forming a brine solution.

For deicing activities, the most common deicing chemical known on the market is sodium chloride, NaCl, which is copiously spread on the roads during the winter with the appearance of the first frosts and snows. Other chloride deicers are also known, for example magnesium chloride in flakes or pellets or liquid form, calcium chloride also in flakes or pellets or liquid form, and complex chloride minerals as variety of minerals mixed, but these chloride salts are very corrosive, especially for automotive corrosion, and bad for the environment through groundwater pollution. For example, it is known from the state of the art document US11066588 disclosing a deicing composition with inorganic salt (25% to 90%), a solvent, lignin (5% to 50%) and a sugar compound (0.1% to 30%).

Homemade deicers for domestic use also exist, easily made at home using a mixture of isopropyl alcohol, warm water and drops of dish soap in a spray bottle to be used mainly on cars, windows, windscreens and car lock.

It is also known for example from document US3096290 a deicing composition consisting of 25 to 95 wt% of propanol, 5 to 50 wt% of ethylene glycol and water up to 100% in which said water content is not exceeding 25%.

Although these easy-to-manufacture and easy-to-use products are still widely used today, alternative and improved solutions have been developed.

Indeed, many factors have to be considered for deicers products, for example the pavement temperature. Rock salt or sodium chloride NaCl ideally works down to -21°C / -6°F but colder pavement temperatures imply slower and not ideal working of a such deicer product, and it is recommended to not use dry rock salt at pavement temperatures below -9°C / 15°F, because the deicing action is too slow.

Thus, to speed up the melting processes is it known to add a liquid deicer to granular products or to formulate deicing liquid formulation. Indeed deicing liquid formulations are much faster acting than granular products.

Several groups of non-chloride deicers exist, which are mainly acetates, formates, glycols, succinates and urea.

Deicers using acetate are mainly sodium acetate, calcium magnesium acetate (CMA) and potassium acetate, these products are marginally corrosive to steel and biodegradable but they react with and corrode zinc and thus affect galvanized steel, they potentially result in anoxic conditions as they break down, require more material relative to rock salt and chloride products to get comparable ice melting and they do not perform as well as chloride products at pavement temperatures below -5 °C / 23 °F. Deicers using acetate and especially CMA have advantage when spread just before a snowstorm but these solutions are generally slower to react than chloride salt deicers and are very dusty, creating potential safety problems during spreading.

Formate deicers, like calcium formate, sodium formate, are well known from the market and state of the art, these products are found to be more effective than acetate deicers regarding dust created by the use of the product itself. Moreover these products are known to remove thin layers of ice that have already formed and prevent new snow and ice from accumulating. Unfortunately calcium formate is found to cause spalling of concrete resulting in damage and the release of dust and debris. For example, it is known from the state of the art documents WO03/033614 disclosing formates and more specifically sodium formate in combination with a corrosion inhibitor namely sodium metasilicate, and US6149833 which discloses the use of sodium formate (30% to 70%) with sodium chloride (30% to 70%) and a corrosion inhibitor (0.1% to 3%).

A conventional product known from the state of the art included in the formates deicers is the product Entry^{®} from the company Synatek LP which is a mixture of potassium formate (45% to 54%), water (40% to 49%) and a proprietary adjuvant blend (6%), this product has features of pH from 7.3 to 7.8, freezing point of -63°F/-53°C and viscosity of 9.6 cP at 20°C.

It is also known from the state of the art, liquid deicer formulations including carbohydrates as additive to increase deicer performance by interference with ice crystal formation, reducing corrosion and increasing sticking to surfaces. For example, it is known from the state of the art, documents WO2013/068299 which discloses the combination of lignin derivative with molasses and a deicing agent.

Finally, liquid deicer formulations based on glycerol/glycol have been developed to not causing increased corrosion in steel and to be effective at average (15°F/-9°C to 32°F/0°C) and cold temperatures (-20°F/-28°C to 15°F/- 9°C) but unfortunately it is reported in the literature that products from the glycerol/glycol deicer category are not effective on ice melting at very cold temperatures (below -20°F/-28°C) added to this the fact that these products are generally highly viscous, especially at reduced/cold/very cold temperatures, and are difficult to use/manage, especially using conventional sprayers. For example, it is known from the state of the art document CA2563638 disclosing a deicing fluid including glycerol, a buffer, an antioxidant/preservative and water.

Unfortunately, although deicers according to prior art exist and offer certain advantages, they would still benefit from improvement.

The present invention encounters to solve at least a part of these drawbacks by providing a deicer and more particularly a deicing liquid formulation intended to be sprayed and for spray application which can be used with simple, common and conventional application devices on the market, avoiding the need for complex applicators.

Another problem to be solved by the present invention is to be able to provide a deicing liquid formulation, that can be easily prepared, stored, transported, stable over time and used on site, including a large variety of surfaces and substrates.

Yet another problem to be solved by the present invention is to provide a deicer effective at average temperatures (15°F/-9°C to 32°F/0°C), at cold temperatures (-20°F/-28°C to 15°F/-9°C) and at very cold temperature (below -20°F/-28°C) in order to provide a product universal, polyvalent and effective for different environmental conditions.

Still another problem to be solved by the present invention is to provide a deicer with at least limited or almost no emissions of dust due to the use of the product itself, and in an even more advantageous manner, to provide a deicing liquid formulation according to the invention allowing in a synergistic manner to both deice snow, ice and frost, and also prevent the emission of dust from the ground, substrate, or surface during and after the deicing phase.

To solve these problems, it is provided according to the present invention, a deicing liquid formulation intended for spray application, comprising from 20 to 60 wt% of potassium formate relative to the liquid formulation, from 5 to 40 wt% of glycerin relative to the liquid formulation and water up to 100 wt%.

As it can be seen, the deicer according to the present invention is a deicing liquid formulation to speed up the melting processes and to act faster than granular products to deice snow, ice and frost.

Moreover, the deicing liquid formulation according to the present invention is a selection of potassium formate from the formate compounds group, in combination with glycerin and water up to 100%, with their respective selected proportion of 20 to 60 wt% for potassium formate and 5 to 40 wt% for glycerin.

This allows the present invention to be simple, easy to prepare, store, transport, with a facility of use on different type of substrates, grounds or surfaces. Furthermore, the selected proportion of compounds of the present invention combine both ease of preparation with ease of spray application, by spraying with conventional and existing sprayers and thus avoiding the need of complex and specific sprayers contrary prior art formulations which are too viscous and requires specific applicators.

Surprisingly, the inventors have developed a deicing liquid formulation according to the present invention with selected proportions of potassium formate and glycerin resulting in a freezing point below average temperatures (15°F/-9°C to 32°F/0°C), preferably below cold temperatures (-20°F/-28°C to 15°F/-9°C), more preferably below very cold temperature (below - 20°F/-28°C), even more preferably below extreme temperature (-63°F/-53°C) and very advantageously below very extreme temperature (-94°F/-70°C). This allowing to provide a polyvalent and eco-friendly deicer according to the invention, active over a wide range of temperatures and climatic conditions, while retaining ease of use with a viscosity that allows spray application by conventional devices.

Finally and even more surprisingly, the deicing liquid formulation formulated by the inventors, with specific selection and combination of potassium formate, glycerin and selected proportions, prevents the emission of dust from the deicer itself, contrary to the prior art, but also prevents the emission of dust from the ground, substrate, surface during the deicing phase and after the drying phase, for example, if a drought occurs in full sunlight during the day.

Other embodiments of the deicing liquid formulation according to the invention are mentioned in the appended claims.

Preferably, the deicing liquid formulation according to the present invention comprises from 25 to 55 wt% of potassium formate, preferably from 25 to 50 wt% of potassium formate, more preferably from 30 to 50 wt% of potassium formate, advantageously from 35 to 50 wt% of potassium formate, in particular from 40 to 50 wt% of potassium formate.

For example, 26 wt%, 27 wt%, 28 wt%, 29 wt%, 30 wt%, 31 wt%, 32 wt%, 33 wt%, 34 wt%, 35 wt%, 36 wt%, 37 wt%, 38 wt%, 39 wt%, 40 wt%, 41 wt%, 42 wt%, 43 wt%, 44 wt%, 45 wt%, 46 wt%, 47 wt%, 48 wt%, 49 wt% of potassium formate.

Advantageously, the deicing liquid formulation according to the present invention comprises from 10 to 40 wt% of glycerin, preferably from 15 to 40 wt% of glycerin, more preferably from 20 to 40 wt% of glycerin, advantageously from 25 to 40 wt% of glycerin.

For example 26 wt%, 27 wt%, 28 wt%, 29 wt%, 30 wt%, 31 wt%, 32 wt%, 33 wt%, 34 wt%, 35 wt%, 36 wt%, 37 wt%, 38 wt%, 39 wt% of glycerin.

In an advantageous embodiment, the deicing liquid formulation according to the present invention comprises a microbial fraction comprising a series of biofilm forming bacteria species.

Preferably, said deicing liquid formulation according to the present invention comprises from 0.1 to 5 wt% of said microbial fraction comprising a series of biofilm forming bacteria species.

For example 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2.0 wt%, 2.1 wt%, 2.2 wt%, 2.3 wt%, 2.4 wt%, 2.5 wt%, 2.6 wt%, 2.7 wt%, 2.8 wt%, 2.9 wt%, 3.0 wt%, 3.1 wt%, 3.2 wt%, 3.3 wt%, 3.4 wt%, 3.5 wt%, 3.6 wt%, 3.7 wt%, 3.8 wt%, 3.9 wt%, 4.0 wt%, 4.1 wt%, 4.2 wt%, 4.3 wt%, 4.4 wt%, 4.5 wt%, 4.6 wt%, 4.7 wt%, 4.8 wt%, 4.9 wt% of said microbial fraction comprising a series of biofilm forming bacteria species
More preferably, said biofilm forming bacteria species of said deicing liquid formulation according to the present invention are chosen in the group comprising Bacillus amyloliquefaciens, Bacillus licheniformis, Bacillus megaterium, Bacillus pumilis, Bacillus subtilis, Bacillus subtilis subspecies Inaquosorum, Bacillus subtilis subspecies subtilis, Bacillus velezensis.

Even more preferably, said series of biofilm forming bacteria species of said deicing liquid formulation according to the present invention contains at least 1 million (1×10⁶) cfu total biofilm forming bacteria per gram of said deicing liquid formulation, preferably at least 5 million (5×10⁶) cfu total biofilm forming bacteria per gram of said deicing liquid formulation, preferably at least 10 million (1×10⁷) cfu total biofilm forming bacteria per gram of said deicing liquid formulation, preferably at least 20 million (2×10⁷) cfu total biofilm forming bacteria per gram of said deicing liquid formulation, more preferably at least 30 million (3×10⁷) cfu total biofilm forming bacteria per gram of said deicing liquid formulation, even more preferably at least 50 million (5×10⁷) cfu total biofilm forming bacteria per gram of said deicing liquid formulation, in particular at least 80 million (8×10⁷) cfu total biofilm forming bacteria per gram of said deicing liquid formulation, more particular at least 100 million (1×10⁸) cfu total biofilm forming bacteria per gram of said deicing liquid formulation, even more particular at least 150 million (1.5×10⁸) cfu total biofilm forming bacteria per gram of said deicing liquid formulation, for example at least 200 million (2×10⁸) cfu total biofilm forming bacteria per gram of said deicing liquid formulation, advantageously at least 250 million (2.5×10⁸) cfu total biofilm forming bacteria per gram of said deicing liquid formulation, more advantageously at least 300 million (3×10⁸) cfu total biofilm forming bacteria per gram of said deicing liquid formulation, even more advantageously at least 400 million (4×10⁸) cfu total biofilm forming bacteria per gram of said deicing liquid formulation, preferably 500 million (5×10⁸) cfu total biofilm forming bacteria per gram of said deicing liquid formulation, preferably contains at least 600 million (6×10⁸), more preferably contains at least 700 million (7×10⁸), even more preferably contains at least 800 million (8×10⁸), advantageously contains at least 900 million (9×10⁸), more advantageously contains at least 1 billion (1×10⁹), preferably contains at least 1.5 billion (1.5×10⁹), more preferably contains at least 2 billion (2×10⁹), even more preferably contains at least 2.5 billion (2.5×10⁹), preferably contains at least 3 billion (3×10⁹), more preferably contains at least 3.5 billion (3.5×10⁹), even more preferably contains at least 4 billion (4×10⁹), advantageously contains at least 4.5 billion (4.5×10⁹), even more preferably contains at least 5 billion (5×10⁹), in particular contains at least 5.5 billion (5.5×10⁹), preferably contains at least 6 billion (6×10⁹), more preferably contains at least 6.5 billion (6.5×10⁹), in particular contains at least 7 billion (7×10⁹), more particularly contains at least 7.5 billion (7.5×10⁹), even more preferably contains at least 8 billion (8×10⁹), preferably contains at least 9 billion (9×10⁹), advantageously contains at least 10 billion (1×10¹⁰) cfu total biofilm forming bacteria per gram of said deicing liquid formulation.

Preferably, said series of biofilm forming bacteria species of said deicing liquid formulation according to the present invention comprises two or more biofilm forming bacteria species, more preferably comprising three or more biofilm forming bacteria species, more preferably comprising four or more biofilm forming bacteria species, even more preferably comprising five or more biofilm forming bacteria species.

More preferably, said series of biofilm forming bacteria species of said deicing liquid formulation according to the present invention contains :
- at least 10 thousand (1×10⁴), preferably at least 20 thousand (2×10⁴), more preferably at least 30 thousand (3×10⁴), even more preferably at least 40 thousand (4×10⁴), in particular at least 50 thousand (5×10⁴), more particular at least 60 thousand (6×10⁴), even more particular at least 70 thousand (7×10⁴), advantageously at least 80 thousand (8×10⁴), more advantageously at least 90 thousand (9×10⁴), preferably at least 0.1 million (1×10⁵), in particular at least 0.5 million (5×10⁵), more particular at least 0.8 million (8×10⁵), preferably at least 1 million (1×10⁶), more preferably at least 2 million (2×10⁶), in particular at least 4 million (4×10⁶), particularly at least 4.7 million (4.7×10⁶), preferably at least 5 million (5×10⁶), more preferably at least 10 million (1x10⁷), in particular at least 20 million (2x10⁷), particularly at least 50 million (5×10⁷) preferably at least 70 million (7×10⁷), more preferably at least 100 million (1×10⁸), particularly at least 150 million (1.5x10⁸) preferably at least 200 million (2×10⁸), more preferably at least 250 million (2.5×10⁸), in particular at least 300 million (3×10⁸), particularly at least 350 million (3.5×10⁸), more preferably at least 400 million (4×10⁸), particularly at least 450 million (4.5×10⁸), more preferably at least 500 million (5×10⁸), cfu of Bacillus licheniformis per gram of said deicing liquid formulation measured according to ASTM-D5465-93 (dated 1998) standard, and/or
- at least 1 million (1×10⁶), preferably at least 2 million (2×10⁶), more preferably at least 3 million (3×10⁶), even more preferably at least 4 million (4×10⁶), in particular at least 5 million (5×10⁶), more particular at least 6 million (6×10⁶), even more particular at least 7 million (7×10⁶), advantageously at least 8 million (8×10⁶), more advantageously at least 9 million (9×10⁶), preferably at least 10 million (1×10⁷), preferably at least 50 million (5×10⁷), more preferably at least 100 million (1×10⁸), in particular at least 200 million (2×10⁸), particularly at least 300 million (3×10⁸), advantageously at least 400 million (4×10⁸), more particular at least 500 million (6×10⁸), in particular at least 700 million (7×10⁸), preferably at least 800 million (8×10⁸), even more preferably at least 900 million (9×10⁸), for example at least 920 million (9.2×10⁸), preferably at least 950 million (9.5×10⁸), more preferably at least 1 billion (1×10⁹), cfu of Bacillus megaterium per gram of said deicing liquid formulation measured according to ASTM-D5465-93 (dated 1998) standard, and/or
- at least 1 thousand (1×10³), preferably at least 2 thousand (2×10³), more preferably at least 3 thousand (3×10³), even more preferably at least 5 thousand (5×10³), in particular at least 10 thousand (1×10⁴), more particular at least 20 thousand (2×10⁴), even more particular at least 50 thousand (5×10⁴), advantageously at least 70 thousand (7×10⁴), more advantageously at least 90 thousand (9×10⁴), preferably at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶), cfu of Bacillus amyloliquefaciens per gram of said deicing liquid formulation measured according to ASTM-D5465-93 (dated 1998) standard, and/or
- at least 10 million (1×10⁷), preferably at least 20 million (2x10⁷), more preferably at least 30 million (3×10⁷), even more preferably at least 50 million (5×10⁷), in particular at least 80 million (8×10⁷), preferably at least 100 million (1×10⁸), particularly at least 250 million (2.5×10⁸) preferably at least 400 million (4×10⁸), more preferably at least 600 million (6×10⁸), in particular at least 750 million (7.5×10⁸), particularly at least 800 million (8×10⁸), more preferably at least 900 million (9×10⁸), in particular at least 1 billion (1×10⁹), preferably at least 2 billion (2×10⁹), more particular 2.8 billion (2.8×10⁹), more preferably at least 3.6 billion (3.6×10⁹), in particular at least 5 billion (5×10⁹), particularly at least 7 billion (7×10⁹), advantageously at least 7.2 billion (7.2×10⁹), more particular at least 8 billion (8×10⁹), particularly at least 8.5 billion (8.5×10⁹), cfu of Bacillus subtilis per gram of said deicing liquid formulation measured according to ASTM-D5465-93 (dated 1998) standard, and/or
- at least 10 million (1×10⁷), preferably at least 20 million (2x10⁷), more preferably at least 30 million (3×10⁷), even more preferably at least 50 million (5×10⁷), in particular at least 80 million (8×10⁷), preferably at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2×10⁹), preferably at least 4 billion (4×10⁹), advantageously at least 5 billion (5×10⁹), more particular at least 7 billion (7×10⁹) cfu of Bacillus subtilis subspecies Inaquosorum per gram of said deicing liquid formulation measured according to ASTM-D5465-93 (dated 1998) standard, and/or
- at least 10 million (1×10⁷), preferably at least 20 million (2×10⁷), more preferably at least 30 million (3×10⁷), even more preferably at least 50 million (5×10⁷), in particular at least 80 million (8×10⁷), preferably at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2×10⁹), preferably at least 4 billion (4×10⁹), advantageously at least 5 billion (5×10⁹), more particular at least 7 billion (7×10⁹) cfu of Bacillus subtilis subspecies subtilis per gram of said deicing liquid formulation measured according to ASTM-D5465-93 (dated 1998) standard, and/or
- at least 1 million (1×10⁶), preferably at least 2 million (2×10⁶), more preferably at least 3 million (3×10⁶), even more preferably at least 4 million (4×10⁶), in particular at least 5 million (5×10⁶), more particular at least 6 million (6×10⁶), even more particular at least 7 million (7×10⁶), advantageously at least 8 million (8×10⁶), more advantageously at least 9 million (9×10⁶), preferably at least 10 million (1×10⁷), preferably at least 50 million (5×10⁷), more particular 100 million (1×10⁸), more preferably at least 200 million (2×10⁸), in particular at least 300 million (3×10⁸), particularly at least 350 million (3.5×10⁸), advantageously at least 400 million (4×10⁸), more particular at least 450 million (4.5×10⁸), even more preferably at least 500 million (5×10⁸) cfu of Bacillus pumilis per gram of said deicing liquid formulation measured according to ASTM-D5465-93 (dated 1998) standard, and/or
- at least 1 thousand (1×10³), preferably at least 2 thousand (2×10³), more preferably at least 3 thousand (3×10³), even more preferably at least 5 thousand (5×10³), in particular at least 10 thousand (1×10⁴), more particular at least 20 thousand (2×10⁴), even more particular at least 50 thousand (5×10⁴), advantageously at least 70 thousand (7×10⁴), more advantageously at least 90 thousand (9×10⁴), preferably at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶) cfu of Bacillus velezensis per gram of said deicing liquid formulation measured according to ASTM-D5465-93 (dated 1998) standard.

In a particularly surprising and advantageous way, the inventors have succeeded in developing a deicing liquid formulation comprising a series of biofilm forming bacteria species, which remains stable over time after preparation, particularly during its storage and transport, and which retains the activity of the bacteria to be able to form a biofilm in order to provide dust suppressant activity.

Moreover, the deicing liquid formulation according to the present invention comprising a series of biofilm forming bacteria species allow a synergistic combination of effects, namely :
- providing an eco-friendly deicer which is polyvalent, active over a wide range of temperatures and climatic conditions,
- retaining ease of use with a viscosity allowing spray application by conventional devices,
- preventing the emission of dust from the deicer itself,
- preventing the emission of dust from the ground, substrate, surface during and after the deicing and drying phases,
- retaining the activity of the bacteria to be able to form a biofilm on the ground/substrate/surface and provide a dust suppressant activity over time.

Furthermore, the specific selection of bacteria from the group of bacteria species and the specific amount of said bacteria, enable the active generation of a biofilm on the ground/substrate/surface that starts growing quite quickly (within 48 hours). This biofilm serves as a protective matrix, providing an ideal habitat for the bacteria to thrive and grow. The biofilm enhances the ability of bacteria to adhere firmly, ensuring maximum coverage and prolonged activity regarding dust suppression.

Thus, the present invention presents a triple and synergistic action, when applied to the substrate/ground/surface to be treated on site, at average, cold, very cold, extreme or very extreme temperatures, first acting immediately as deicer, second the generation of liquid from ice reduce the ability of dust particles to become airborne, and third the bacteria actively generate a biofilm on the substrate/ground/surface, when conditions allow the formation of a biofilm, thus enhancing their ability to adhere firmly and ensuring maximum coverage and prolonged activity to suppress dust emissions over time.

Consequently, the present invention is a deicing and dust-suppressant liquid formulation.

Moreover, it turns out that when two, three, four or even five or more different species of bacteria of the present invention are present, the ability to form a biofilm and the dust suppressant activity are improved, when conditions allow.

Preferably, the deicing liquid formulation according to the present invention comprises at least a binder chosen from the group comprising a latex binder, lignosulfonate, xanthan gum, acacia gum, cellulose and the like, their derivatives and their combination.

In a preferred embodiment, said latex binder is a biobased latex binder, in particular a biobased latex binder having surfactant properties, more preferably a biobased starch latex binder, more particular a biobased modified starch latex binder.

More preferably, the deicing liquid formulation according to the present invention comprises one or more additive chosen in the group comprising corrosion inhibitors, humectants, viscosity modifiers, preservatives, surfactants, dispersants, electrolytes, emulsifying agents, pH modifiers, pigments and their combination thereof.

Preferably, the deicing liquid formulation according to the present invention presents a freezing point below -53°C, more preferably below -55°C, even more preferably below -57°C, advantageously below -60°C, more advantageously below -63°C, even more advantageously below -65°C, in particular below -68°C, more particular below -70°C.

In a preferred embodiment, the deicing liquid formulation according to the present invention presents a pH between 4 and 8.

This allowing to the present invention, due to the presence of the binder, for example the latex binder and/or the lignosulfonate and/or other additives (xanthan gum, acacia gum, cellulose and the like, their derivates and their combination) as a food source and carbon supply for said series of dust suppressing bacteria species, the bacteria actively generate a biofilm on the ground/substrate/surface enhancing their ability to adhere firmly and ensuring maximum coverage and prolonged activity to suppress dust emissions, when conditions allow.

Moreover, the surfactant properties of the biobased latex binder enhance its effectiveness in solubilizing bacteria in aqueous phases and in the deicing liquid formulation according to the invention. Surfactants are surface-active agents that reduce the surface tension between liquids, enabling better mixing and dispersion of the binder within the aqueous medium. When used for solubilizing bacteria, the surfactant properties of the biobased latex binder promote faster and more efficient emulsification, creating a homogenous mixture with the bacteria. This results in quicker solubilization, saving valuable time. Furthermore, the surfactant nature of the biobased latex binder enhances its ability to stabilize the solubilized bacteria over time. By forming micelles or stabilizing the interface between the bacteria and the aqueous phase, it helps prevent aggregation or precipitation, ensuring the integrity of the bacterial solution even during extended storage periods. In addition, starch latex binders have excellent biodegradability, allowing them to naturally break down over time without leaving harmful residues. This characteristic is beneficial to reduce waste and environmental pollution. Further, one significant advantage of a biobased modified starch latex binder lies in its enhanced performance compared to traditional starch binders. Through modification, the binder gains improved stability, versatility, and adhesive properties. This results in stronger and more durable bonding.

Other embodiments of the deicing liquid formulation of the present invention are mentioned in the appended claims.

The present invention also relates to an utilization of the deicing liquid formulation intended for spray application according to the invention, to suppress ice on a substrate, for example on roads, mining roads, conveyers, private roads, public roads, construction sites, roadways, highways, parking, walkways, bridges, cars, trucks including their windows and windscreens, trains and rail switches, aircrafts, airport pavement including runways, taxiways, airport aprons and taxiway bridges, at home and for domestic use, like on sidewalks and driveways.

More particularly, said utilization of the deicing liquid formulation intended for spray application according to the invention, to suppress ice, snow, frost on a substrate, for example roads, mining roads, conveyers, private roads, public roads, walkways, public walkways, industrial walkways, parking areas, ramps, domestic sidewalks and driveways, construction sites, industrial work areas.

Other embodiments of the utilization of said deicing liquid formulation of the present invention are mentioned in the appended claims.

Other characteristics and advantages of the present invention will be derived from the non-limitative following description, and by making reference to the examples.

### Examples.-

### Round 1 of Examples :

Several deicing liquid formulations have been prepared according to the Table 1 below.

**Table 1**

| Formulations | Latex binder | Biofilm forming bacteria | Glycerin | Potassium formate | Water |
|---|---|---|---|---|---|
| Comparative Formulation 1 without Potassium formate | | 0.93% | 79% | | 20.09% |
| Comparative Formulation 2 without Biofilm forming bacteria | 0.93% | | 79% | | 20.09% |
| Deicer-1 according to the present invention | | 0.93% | 39% | 30% | 30% |
| Deicer-2 according to the present invention | | 0.93% | 29% | 35% | 35% |
| Deicer-3 according to the present invention | | | 30% | 35% | 35% |
| Glycerin | | | 100% | | |
| Commercial product - Potassium formate based product | Used "as is" | | | | |

Formulation Glycerin from Table 1 became viscous to thick syrup conditions at a freezing temperature below -5°C, making application of this formulation impossible using conventional spray device/bottle/applicators. Therefore, this formulation was excluded from further evaluations.

### Methodology:

### Ice melting capacity:

The ice melting capacity test procedure was developed to evaluate the ability of each test formulation to melt ice in a glass vial.

According to the procedure, 40 ml of tap water was placed to the Pyrex glass dish and stored in the freezer until thick layer of ice (5-7mm) is formed. Prepared test formulations and syringes were stored in the freezer as well. When water in glass container was completely frozen, 1 ml of test formulation was applied on the surface of the ice by syringe, and the glass containers were replaced to the freezer. Every 15 minutes after application glass containers were taken out of the freezer one by one and volume of the liquid released was measured by dedicated syringes.

Performance of the formulations according to Table 1 was evaluated based on volume of the liquid collected from the surface of the ice and time during which the deicer maintained liquid on the surface of the ice. The ice melting capacity is a function of the maximum volume of liquid created and the duration of the liquid phase before eventually freezing again.

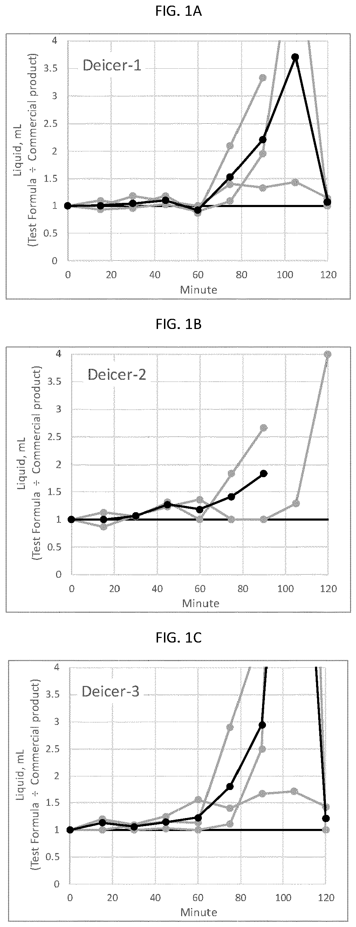

Figure 1A, Figure 1B & Figure 1C represent the ice melting capacity of respectively formulations Deicer 1 according to the present invention, Deicer 2 according to the present invention & Deicer 3 according to the present invention, in comparison to the Commercial product - Potassium formate based prod uct.

On Figures 1A, 1B & 1C, when the lines in grey are higher than 1, there is more liquid in the Deicer formulations 1, 2 and 3 according to the invention compared to the Commercial product. When the lines in grey are lower than 1, it indicates less liquid (and thus more freezing) in the test formula vs. the Commercial product. Average curve of formulations is provided in black.

It appears that Deicer formulations 1, 2 and 3 according to the invention performed regarding ice melting capacity as well or better than the Commercial product.

The effect was seen with quick increases in the amount of liquid early in the test compared to the Commercial product, and the melting effect lasted longer than the Commercial product, with more liquid in the samples after 90 - 105 minutes.

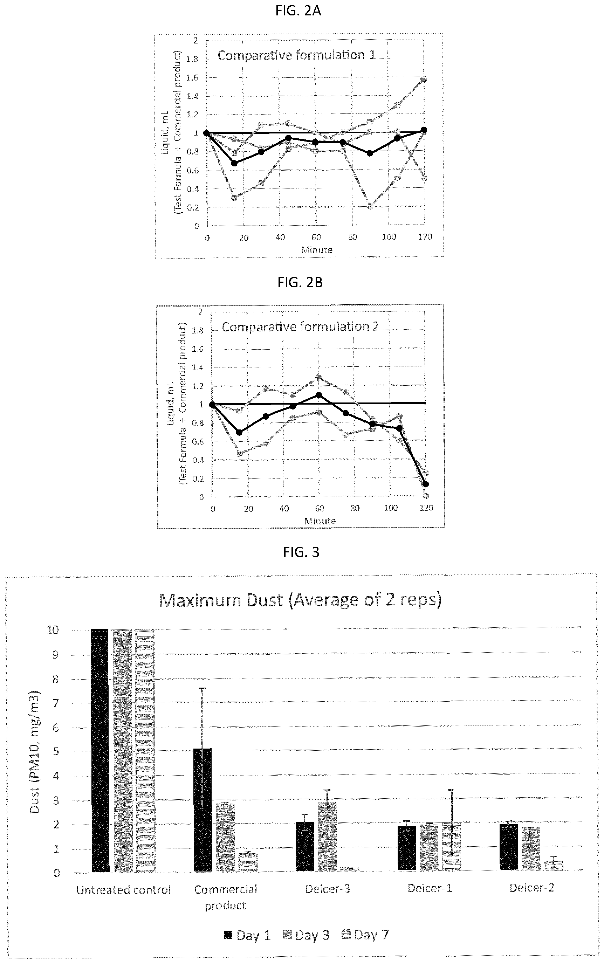

Figure 2A and Figure 2B are comparative test, representing ice melting capacity of respectively Comparative Formulation 1 without Potassium formate and Comparative Formulation 2 without Biofilm forming bacteria, in comparison to the Commercial product - Potassium formate based product.

On Figures 2A and 2B, when the lines in grey are higher than 1, there is more liquid in the Comparative Formulation 1 and/or 2 not from the invention compared to the Commercial product. When the lines in grey are below than 1, it indicates less liquid (and thus more freezing) in the test formula vs. the Commercial product. Average curve of formulations is provided in black.

It appears that Comparative Formulation 1 and 2 not from the invention were less efficient regarding ice melting capacity than the Commercial prod uct.

The samples generally started solidifying (freezing) more quickly than the Commercial product, and produced less liquid so thus a less-strong deicing effect.

### Formulation freezing point:

Differential scanning calorimetry (DSC) is a typical method to determine freezing point of material. To perform the test, a sample is placed in the DSC calorimeter. Temperature is gradually reduced from room temperature to -70 or -80°C. The temperature at which the deicer/tested formulation begins to freeze is marked by a sharp peak or dip on the thermogram. This peak strongly correlates to the temperature at which a particular concentration of deicer/tested formulation will remain effective.

The freezing point of liquid deicers is an important parameter defining the functional operating conditions for different formulations.

According to the product's technical documentation, the freezing point of the Commercial product - Potassium formate based product is -53°C.

Deicer-2 according to the present invention and Commercial product - Potassium formate based product - Formulations from **Error! Reference source not found,** were submitted to DSC analysis at NanoQAM centre to determine their freezing points. Equipment used in the center is capable to run samples until -70°C. As a control a sample of n-octane (freezing point=-57°C) was used.

Interestingly, none of the two formulations froze during the test, leading to the conclusion that these formulations have freezing points below -70°C.

Moreover, Deicer-2 formulation according to the present invention is considered at equivalent regarding the freezing point feature than the commercial potassium formate based product, but the formulation according to the invention imply less amount of potassium formate and is more ecological.

Furthermore, the deicing liquid formulations according to the present invention with selected proportions of potassium formate and glycerin resulting in a freezing point below average temperatures (15°F/-9°C to 32°F/0°C), preferably below cold temperatures (-20°F/-28°C to 15°F/-9°C), more preferably below very cold temperature (below -20°F/-28°C), even more preferably below extreme temperature (-63°F/-53°C) and very advantageously below very extreme temperature (-94°F/-70°C).

### Dust suppressant properties :

Stone dust samples were treated with formulations according to Table 1 :
- Commercial product - Potassium formate-based product,
- Deicer-1 formulation,
- Deicer-2 formulation,
- Deicer-3 formulation,
and stored at room temperature. Treated samples were tested using the drop test method at days 1, 3, 7 (Figure 3).

Dust emissions from each sample were evaluated by dropping **25** grams of material in a closed chamber while an attached dust monitor logged airborne particulate over time. The tests were performed **day 1, day 3 and day 7** after treatment.

The Drop test methodology consists of:
1. Measure out 25 grams of each treated sample into clean labelled dishes.
2. Process each sample one at a time using the following drop test procedure:
   a. Connect the DustTrak DRX Aerosol Monitor 8533 to the drop cylinder using the flexible hose (Figure 3).
   b. Set the DustTrak RunMode to 2-minute duration, with 1 second sampling frequency.
   c. Start the DustTrak logging.
   d. Wait 30 seconds to allow readings to settle to a consistent background level.
   e. Lift the top cover of the cylinder, quickly drop the sample material into the cylinder and replace the top cover.
   f. Allow the DustTrack to run for the full 2-minute duration.
   g. Disconnect the DustTrack, clean the drop cylinder using cloths and anti-static sheets.
   h. Discard the dropped sample material and clean the bottom plate.
   i. Replace equipment and proceed to the next sample.
3. Import the data into Excel, calculate the maximum dust level recorded for each sample and produce summary statistics.

As it can be seen from Fig. 3, untreated sample showed the highest level of dust on all days (with a value of 149 mg/m3).

All treated samples were able to control the dust at relatively similar levels. Moreover, Deicer formulations 1, 2 and 3 according to the present invention, all controlled dust at similar levels and showed lower levels of dust compared to the Commercial product.

Furthermore, Deicer formulations 1 and 2 - which comprise biofilm forming bacteria, provided an additional effect on dust suppression on day 3, compared with the Deicer formulation 3 according to invention not comprising biofilm forming bacteria.

### Round 2 of Examples :

The round 2 examples were designed to generate additional information about the deicing strength of different combinations of formulations according to the present invention.

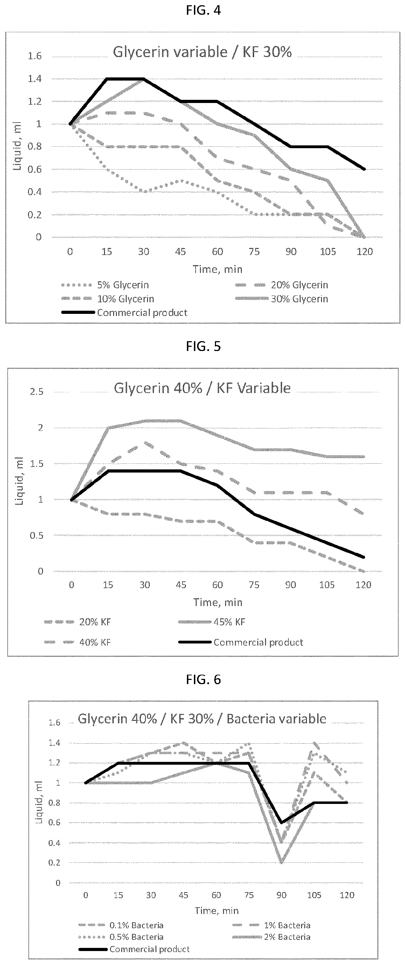

Figure 4 illustrates the ice melting capacity (production of liquid from ice overtime, with same methodology as presented above) of formulations according to the present invention in which potassium formate was held constant at 30 wt%, while glycerin levels varied from 5% to 30%, in comparison to the Commercial Product of Table 1.

It is shown that, in Figure 4, formulations according to the present invention present ice melting capacity at each level of glycerin 5 wt%, 10 wt%, 20 wt% and 30 wt%, with equivalent ice melting capacity to the Commercial product tested for formulation according to the invention with an amount above 20 wt% of glycerin.

Figure 5 illustrates the ice melting capacity (production of liquid from ice overtime, with same methodology as presented above) of formulations according to the present invention in which glycerin was held constant at 40 wt%, while potassium formate levels varied from 20% to 45%, in comparison to the Commercial Product of Table 1.

It is shown that, in Figure 5, formulations according to the present invention present ice melting capacity at each level of potassium formate 20 wt%, 40 wt% and 45 wt%, with better ice melting capacity than the Commercial product tested for formulation according to the invention with an amount equal to or above 40 wt% of potassium formate

Figure 6 illustrates the ice melting capacity (production of liquid from ice overtime, with same methodology as presented above) of formulations according to the present invention in which glycerin was held constant at 40 wt%, and potassium formate was held constant at 30%, and in which biofilm forming bacteria mixed with binding agent as wettable powder were added at four different concentrations, in comparison to the Commercial Product of Table 1.

It is shown that, in Figure 6, formulations according to the present invention present ice melting capacity at each level of biofilm forming bacteria 0.1 wt%, 0.5 wt%, 1 wt% and 2 wt%, with equivalent ice melting capacity than the Commercial product tested.

Moreover, formulations according to the present invention are considered as equivalent regarding the ice melting capacity of the commercial potassium formate based product, but the formulations according to the invention comprising a lower concentration of potassium formate, are more ecological and provide a synergistic dust suppressant effect over time.

Thus, formulations tested according to the present invention, advantageously can produce similar or even superior deicing effects compared to commercially available product based on potassium formate (with higher concentration of potassium formate).

It should be understood that the present invention is not limited to the described embodiments and that variations can be applied without going outside of the scope of the appended claims.

## Claims

1. Deicing liquid formulation intended for spray application, comprising from 20 to 60 wt% of potassium formate relative to the liquid formulation, from 5 to 40 wt% of glycerin relative to the liquid formulation and water up to 100 wt%.

2. Deicing liquid formulation intended for spray application according to claim 1, comprising from 25 to 55 wt% of potassium formate, preferably from 25 to 50 wt% of potassium formate, more preferably from 30 to 50 wt% of potassium formate, advantageously from 35 to 50 wt% of potassium formate, in particular from 40 to 50 wt% of potassium formate.

3. Deicing liquid formulation intended for spray application according to claim 1 or 2, comprising from 10 to 40 wt% of glycerin, preferably from 15 to 40 wt% of glycerin, more preferably from 20 to 40 wt% of glycerin, advantageously from 25 to 40 wt% of glycerin.

4. Deicing liquid formulation intended for spray application according to claim 1 to 3, comprising a microbial fraction comprising a series of biofilm forming bacteria species.

5. Deicing liquid formulation intended for spray application according to claim 4, comprising from 0.1 to 5 wt% of said microbial fraction comprising a series of biofilm forming bacteria species.

6. Deicing liquid formulation intended for spray application according to claim 4 or 5, wherein said biofilm forming bacteria species are chosen in the group comprising Bacillus amyloliquefaciens, Bacillus licheniformis, Bacillus megaterium, Bacillus pumilis, Bacillus subtilis, Bacillus subtilis subspecies Inaquosorum, Bacillus subtilis subspecies subtilis, Bacillus velezensis.

7. Deicing liquid formulation intended for spray application according to claim 4 to 6, wherein said series of biofilm forming bacteria species contains at least 1 million (1×10⁶) cfu total biofilm forming bacteria per gram of said deicing liquid formulation, preferably at least 5 million (5×10⁶) cfu total biofilm forming bacteria per gram of said deicing liquid formulation, preferably at least 10 million (1×10⁷) cfu total biofilm forming bacteria per gram of said deicing liquid formulation, preferably at least 20 million (2×10⁷) cfu total biofilm forming bacteria per gram of said deicing liquid formulation, more preferably at least 30 million (3×10⁷) cfu total biofilm forming bacteria per gram of said deicing liquid formulation, even more preferably at least 50 million (5×10⁷) cfu total biofilm forming bacteria pergram of said deicing liquid formulation, in particular at least 80 million (8×10⁷) cfu total biofilm forming bacteria per gram of said deicing liquid formulation, more particular at least 100 million (1×10⁸) cfu total biofilm forming bacteria per gram of said deicing liquid formulation, even more particular at least 150 million (1.5×10⁸) cfu total biofilm forming bacteria per gram of said deicing liquid formulation, for example at least 200 million (2×10⁸) cfu total biofilm forming bacteria per gram of said deicing liquid formulation, advantageously at least 250 million (2.5×10⁸) cfu total biofilm forming bacteria per gram of said deicing liquid formulation, more advantageously at least 300 million (3×10⁸) cfu total biofilm forming bacteria per gram of said deicing liquid formulation, even more advantageously at least 400 million (4×10⁸) cfu total biofilm forming bacteria per gram of said deicing liquid formulation, preferably at least 500 million (5×10⁸) cfu total biofilm forming bacteria per gram of said deicing liquid formulation, preferably contains at least 600 million (6×10⁸), more preferably contains at least 700 million (7×10⁸), even more preferably contains at least 800 million (8×10⁸), advantageously contains at least 900 million (9×10⁸), more advantageously contains at least 1 billion (1×10⁹), preferably contains at least 1.5 billion (1.5×10⁹), more preferably contains at least 2 billion (2×10⁹), even more preferably contains at least 2.5 billion (2.5×10⁹), preferably contains at least 3 billion (3×10⁹), more preferably contains at least 3.5 billion (3.5×10⁹), even more preferably contains at least 4 billion (4×10⁹), advantageously contains at least 4.5 billion (4.5×10⁹), even more preferably contains at least 5 billion (5×10⁹), in particular contains at least 5.5 billion (5.5×10⁹), preferably contains at least 6 billion (6×10⁹), more preferably contains at least 6.5 billion (6.5×10⁹), in particular contains at least 7 billion (7×10⁹), more particularly contains at least 7.5 billion (7.5×10⁹), even more preferably contains at least 8 billion (8×10⁹), preferably contains at least 9 billion (9×10⁹), advantageously contains at least 10 billion (1×10¹⁰) cfu total biofilm forming bacteria per gram of said deicing liquid formulation.

8. Deicing liquid formulation intended for spray application according to claim 6 or 7, wherein said series of biofilm forming bacteria species comprises two or more biofilm forming bacteria species, more preferably comprising three or more biofilm forming bacteria species, more preferably comprising four or more biofilm forming bacteria species, even more preferably comprising five or more biofilm forming bacteria species.

9. Deicing liquid formulation intended for spray application according to claims 4 to 8, wherein said series of biofilm forming bacteria species contains :
- at least 10 thousand (1×10⁴), preferably at least 20 thousand (2×10⁴), more preferably at least 30 thousand (3×10⁴), even more preferably at least 40 thousand (4×10⁴), in particular at least 50 thousand (5×10⁴), more particular at least 60 thousand (6×10⁴), even more particular at least 70 thousand (7×10⁴), advantageously at least 80 thousand (8×10⁴), more advantageously at least 90 thousand (9×10⁴), preferably at least 0.1 million (1×10⁵), in particular at least 0.5 million (5×10⁵), more particular at least 0.8 million (8×10⁵), preferably at least 1 million (1×10⁶), more preferably at least 2 million (2×10⁶), in particular at least 4 million (4×10⁶), particularly at least 4.7 million (4.7×10⁶), preferably at least 5 million (5×10⁶), more preferably at least 10 million (1×10⁷), in particular at least 20 million (2x10⁷), particularly at least 50 million (5×10⁷) preferably at least 70 million (7×10⁷), more preferably at least 100 million (1×10⁸), particularly at least 150 million (1.5x10⁸) preferably at least 200 million (2×10⁸), more preferably at least 250 million (2.5×10⁸), in particular at least 300 million (3×10⁸), particularly at least 350 million (3.5×10⁸), more preferably at least 400 million (4×10⁸), particularly at least 450 million (4.5×10⁸), more preferably at least 500 million (5×10⁸), cfu of Bacillus licheniformis per gram of said deicing liquid formulation measured according to ASTM-D5465-93 (dated 1998) standard, and/or
- at least 1 million (1×10⁶), preferably at least 2 million (2×10⁶), more preferably at least 3 million (3×10⁶), even more preferably at least 4 million (4×10⁶), in particular at least 5 million (5×10⁶), more particular at least 6 million (6×10⁶), even more particular at least 7 million (7×10⁶), advantageously at least 8 million (8×10⁶), more advantageously at least 9 million (9×10⁶), preferably at least 10 million (1×10⁷), preferably at least 50 million (5×10⁷), more preferably at least 100 million (1×10⁸), in particular at least 200 million (2×10⁸), particularly at least 300 million (3×10⁸), advantageously at least 400 million (4×10⁸), more particular at least 500 million (6×10⁸), in particular at least 700 million (7×10⁸), preferably at least 800 million (8×10⁸), even more preferably at least 900 million (9×10⁸), for example at least 920 million (9.2×10⁸), preferably at least 950 million (9.5×10⁸), more preferably at least 1 billion (1×10⁹), cfu of Bacillus megaterium per gram of said deicing liquid formulation measured according to ASTM-D5465-93 (dated 1998) standard, and/or
- at least 1 thousand (1×10³), preferably at least 2 thousand (2×10³), more preferably at least 3 thousand (3×10³), even more preferably at least 5 thousand (5×10³), in particular at least 10 thousand (1×10⁴), more particular at least 20 thousand (2×10⁴), even more particular at least 50 thousand (5×10⁴), advantageously at least 70 thousand (7×10⁴), more advantageously at least 90 thousand (9×10⁴), preferably at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶), cfu of Bacillus amyloliquefaciens per gram of said deicing liquid formulation measured according to ASTM-D5465-93 (dated 1998) standard, and/or
- at least 10 million (1×10⁷), preferably at least 20 million (2x10⁷), more preferably at least 30 million (3×10⁷), even more preferably at least 50 million (5×10⁷), in particular at least 80 million (8×10⁷), preferably at least 100 million (1×10⁸), particularly at least 250 million (2.5×10⁸) preferably at least 400 million (4×10⁸), more preferably at least 600 million (6×10⁸), in particular at least 750 million (7.5×10⁸), particularly at least 800 million (8×10⁸), more preferably at least 900 million (9×10⁸), in particular at least 1 billion (1×10⁹), preferably at least 2 billion (2×10⁹), more particular 2.8 billion (2.8×10⁹), more preferably at least 3.6 billion (3.6×10⁹), in particular at least 5 billion (5×10⁹), particularly at least 7 billion (7×10⁹), advantageously at least 7.2 billion (7.2×10⁹), more particular at least 8 billion (8×10⁹), particularly at least 8.5 billion (8.5×10⁹), cfu of Bacillus subtilis per gram of said deicing liquid formulation measured according to ASTM-D5465-93 (dated 1998) standard, and/or
- at least 10 million (1×10⁷), preferably at least 20 million (2x10⁷), more preferably at least 30 million (3×10⁷), even more preferably at least 50 million (5×10⁷), in particular at least 80 million (8×10⁷), preferably at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2×10⁹), preferably at least 4 billion (4×10⁹), advantageously at least 5 billion (5×10⁹), more particular at least 7 billion (7×10⁹) cfu of Bacillus subtilis subspecies Inaquosorum per gram of said deicing liquid formulation measured according to ASTM-D5465-93 (dated 1998) standard, and/or
- at least 10 million (1×10⁷), preferably at least 20 million (2x10⁷), more preferably at least 30 million (3×10⁷), even more preferably at least 50 million (5×10⁷), in particular at least 80 million (8×10⁷), preferably at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2×10⁹), preferably at least 4 billion (4×10⁹), advantageously at least 5 billion (5×10⁹), more particular at least 7 billion (7×10⁹) cfu of Bacillus subtilis subspecies subtilis per gram of said deicing liquid formulation measured according to ASTM-D5465-93 (dated 1998) standard, and/or
- at least 1 million (1×10⁶), preferably at least 2 million (2×10⁶), more preferably at least 3 million (3×10⁶), even more preferably at least 4 million (4×10⁶), in particular at least 5 million (5×10⁶), more particular at least 6 million (6×10⁶), even more particular at least 7 million (7×10⁶), advantageously at least 8 million (8×10⁶), more advantageously at least 9 million (9×10⁶), preferably at least 10 million (1×10⁷), preferably at least 50 million (5×10⁷), more particular 100 million (1×10⁸), more preferably at least 200 million (2×10⁸), in particular at least 300 million (3×10⁸), particularly at least 350 million (3.5×10⁸), advantageously at least 400 million (4×10⁸), more particular at least 450 million (4.5×10⁸), even more preferably at least 500 million (5×10⁸) cfu of Bacillus pumilis per gram of said deicing liquid formulation measured according to ASTM-D5465-93 (dated 1998) standard, and/or
- at least 1 thousand (1×10³), preferably at least 2 thousand (2×10³), more preferably at least 3 thousand (3×10³), even more preferably at least 5 thousand (5×10³), in particular at least 10 thousand (1×10⁴), more particular at least 20 thousand (2×10⁴), even more particular at least 50 thousand (5×10⁴), advantageously at least 70 thousand (7×10⁴), more advantageously at least 90 thousand (9×10⁴), preferably at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶) cfu of Bacillus velezensis per gram of said deicing liquid formulation measured according to ASTM-D5465-93 (dated 1998) standard.

10. Deicing liquid formulation intended for spray application according to claim 1 to 9, comprising at least a binder chosen from the group comprising a latex binder, lignosulfonate, xanthan gum, acacia gum, cellulose and the like, their derivatives and their combination.

11. Deicing liquid formulation intended for spray application according to claim 1 to 10, comprising one or more additive chosen in the group comprising corrosion inhibitor, humectants, viscosity modifiers, preservatives, surfactants, dispersants, electrolytes, emulsifying agents, pH modifiers, pigments and their combination thereof.

12. Method for deicing, comprising the step of applying the deicing liquid formulation intended for spray application according to claims 1 to 11, to a substrate, preferably the applying step is repeated more than once, more preferably twice, even more preferably three times.

13. Utilization of the deicing liquid formulation intended forspray application according to claims 1 to 11, to suppress ice on a substrate, for example on roads, mining roads, conveyers, private roads, public roads, walkways, public walkways, industrial walkways, parking areas, ramps, domestic sidewalks and driveways, construction sites, industrial work areas.
